# EUROPEAN PATENT APPLICATION

(11) **EP 1 767 225 A1**
(43) Date of publication of application: **28.03.2007**
(21) Application number: 05255825.1
(22) Date of filing: 21.09.2005
(51) Int. Cl.: A61L 2/00, A61L 2/10

(54) **Method for stabilisation of a protein solution by addition of hydroxyl radical quenchers and its sterilisation by ionising radiation**

(71) Applicant: Insense Limited, Sharnbrook, Bedford MK44 1LQ (GB)
(72) Inventor: The designation of the inventor has not yet been filed
(74) Representative: Perry, Robert Edward

(57) **Abstract**

A method of sterilising a protein in an aqueous environment, comprises (a) bringing the protein into contact with a protective compound or combination of protective compounds having the following characteristics:
(i) a rate of reaction with hydroxyl radicals greater than 5 x 10⁸ L mol⁻¹ s⁻¹; and
(ii) a non-polar region,
excluding the case where the protein is an enzyme and the protective compound(s) comprise a source of lactate ions and a source of zinc ions and/or a source of ammonium ions and excluding guanine and histidine as protective compounds; and (b) exposing the protein and protective compound(s) to ionising radiation. The invention also provides a method of stabilising a protein in an aqueous environment during sterilisation by exposure to ionising radiation, and a composition comprising protein in an aqueous environment.

## Description

### 1. Field of the invention

This invention relates to the stability of proteins, particularly the stability of proteins in an aqueous environment, for example in aqueous solution, in aqueous gel form or in non-liquid state such as solid state where free or bound water is present e.g. in frozen condition or following partial removal of water such as by freeze-drying.

### 2. Background to the invention

Many proteins, eg enzymes, are unstable and are susceptible to degradation and consequent loss of activity under certain conditions. This applies particularly to proteins in an aqueous environment.

Particular difficulties arise where the protein is required in sterile condition. One effective sterilisation technique involves exposure to ionising radiation, eg gamma radiation, electron beam radiation or X-ray radiation. Sterilisation by exposure to ionising radiation is a particularly aggressive process, typically requiring a composition to be subjected to high doses of ionising radiation, generally in the region of 25 to 40 kGy. These conditions are damaging to proteins, particularly enzymes at dilute working strength (typically 1 µg/ml to 10 mg/ml) and/or to enzymes which are not in immobilised condition for example by being irreversibly bound to a solid support.

WO 2004/108917 discloses protection of enzymes, particularly in hydrated condition, on exposure to sterilising ionising radiation, by contact with a source of lactate ions and a source of zinc ions and/or a source of ammonium ions, e.g. in the form of zinc L-lactate.

JP 06-284886 discloses various combinations of enzymes with particular stabilising materials to improve storage stability, possibly on exposure to heat. Disclosed combinations include bilirubin oxidase stabilised by tryptophan, lactate dehydrogenase stabilised by malic acid salt and lactate dehydrogenase stabilised by succinic acid salt. There is no reference to stability of proteins on exposure to ionising radiation.

The present invention is based on an analysis of the effects of ionising radiation and the development of a model based on this analysis that enables selection of a compound or combination of compounds capable of protecting a protein in aqueous environment against the effects of ionising radiation. The model is presented using gamma radiation as an example of ionising radiation.

### 3. Formulation and discussion of the model

### 3.1 Gamma radiation

Gamma radiation is one of several types of high-energy ionising radiation. It consists of high energy photons that are emitted by nuclei of radioactive atoms (e.g. Cobalt 60). The chemical and biological effects of ionising radiation originate from two basic types of interactions. For direct action, the radiation energy is deposited directly in target molecules. For indirect action, the initial absorption of energy is by the external medium, leading to the production of diffusive intermediates which then attack the targets. It is predominantly the indirect action that causes damage to chemical species dissolved in water. This means that the radiation first interacts with the solvent (i.e. water) to give rise to various free radicals. The free radicals subsequently react with the dissolved species (e.g. proteins). Thus, in order to protect the dissolved species against the effects of gamma rays it is necessary to mitigate the adverse effects of free radicals.

Irradiation of water by gamma irradiation results in formation of three main types of free radical:
- Hydrated electron (e⁻_{aq})·. This is the strongest known reducing species with a standard reduction potential E'₀ = -2.9 V.
- Hydroxyl radical, OH·. This is a very strong oxidising radical.
- Hydrogen radical, H·. This is an oxidising radical that is weaker than hydroxyl radical.

The importance of the hydrogen radical is relatively small compared with that of the hydrated electron and the hydroxyl radical for the following reasons. Firstly, the effects of the hydrogen radical on dissolved species are qualitatively similar to those of hydroxyl radical, but not as strong. Secondly, the yield of hydrogen radical formation in irradiated water is considerably smaller compared with that of hydroxyl radical or hydrated electron. It can therefore be concluded that the main two radicals that will react with dissolved species in solutions subjected to ionising radiation are hydrated electron and hydroxyl radical.

### 3.2 Identification of the susceptible sites of protein molecules for free radical damage

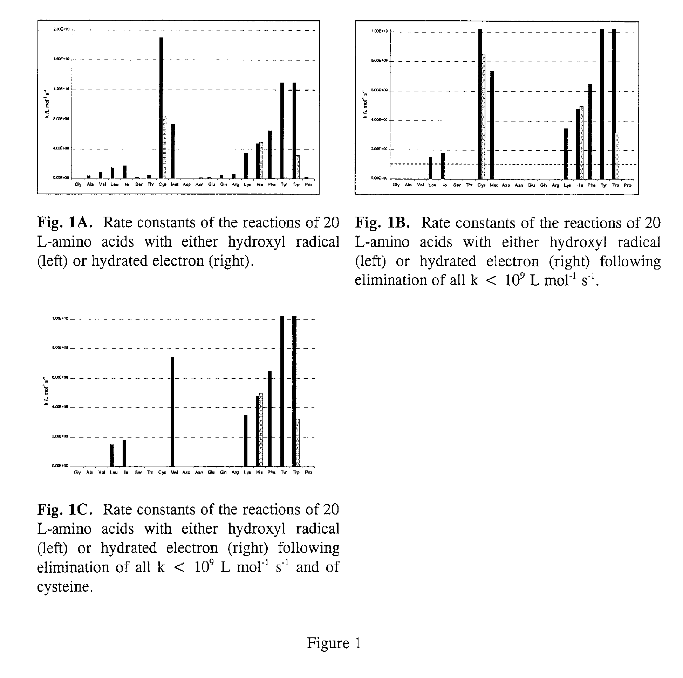

Proteins are macromolecules consisting of sequences of 20 different amino acids. Each of the amino acid units within a protein is theoretically capable of reaction with hydrated electrons or hydroxyl radicals.
The rate constants at ambient temperature (25°C) for the reactions of amino acids with the two free radicals of interest, namely hydrated electron and hydroxyl radical, are shown in Fig. 1A. The rate constant values were obtained from websites maintained by the Radiation Chemistry Data Center (RCDC) of the Notre Dame Radiation Laboratory, an information resource dedicated to the collection, evaluation, and dissemination of data characterizing the reactions of transient intermediates produced by radiation chemical and photochemical methods, as follows:
http://www.rcdc.nd.edu/compilations/Hydroxyl/OH.htm and
http://www.rcdc.nd.edu/compilations/Eaq/Eaq.htm.

Although these rate constant values were for free amino acids, they nevertheless reflect the reactivity of the amino acid when incorporated into a protein. Examination of these data reveal that, of the two major free radicals that are formed in gamma-irradiated aqueous solutions, the hydroxyl radical is considerably more likely to react with the amino acid residues of proteins than the hydrated electron, and thus be responsible for most damage to the protein.

Protection against the effects of hydroxyl radical attack is therefore crucial. Protection against the hydrated electron is also believed to be important, but its significance is secondary compared with that of the hydroxyl radical.

Fig. 1A also shows that there are considerable differences between the individual amino acids in terms of their reaction rates with hydroxyl radicals and hydrated electrons. A reaction rate threshold of 10⁹ L mol⁻¹ s⁻¹ was chosen arbitrarily to eliminate amino acids with "low" rate of reaction with the two free radicals (i.e. amino acids that are not very likely to be attacked by the free radicals). The reaction rates of the amino acids remaining after such elimination are shown in Fig. 1B.

A further simplification of the set can be implemented in the case of most proteins: The relative abundance of cysteine in proteins is very low. Furthermore, cysteine residues are typically engaged in di-sulphide bridges deep inside the protein sub-units. For example, there are only 3 cysteines in each of the glucose oxidase subunits, two of which are engaged in a disulphide bridge deep inside the subunit (cf. 15 lysines, 20 histidines and 28 tyrosines). Cysteine can therefore be also eliminated from the list of amino acids likely to be targeted by the free radicals (Fig. 1C).

Thus, the protein amino acids most likely to be targeted by the hydroxyl radicals and hydrated electrons produced by ionising radiation in aqueous media, are leucine, isoleucine, phenylalanine, tryptophan, methionine, histidine, tyrosine and lysine (see Fig. 1C). Whilst all of these amino acids exhibit a high rate of reaction with hydroxyl radicals, histidine and tryptophan also have a high reaction rate with hydrated electrons.

### 3.3 Interaction of protecting compounds with protein molecule

Degradation of biological systems by reaction with free radical species is well known, and has been associated with many forms of tissue damage, disease and with the process of aging. Protection from free radical damage can be effected using sacrificial molecules that react with and thereby "scavenge" the free radicals. As many free radicals are reactive forms of oxygen, effective scavenger compounds are often referred to as antioxidants. Typical antioxidants include vitamin C (ascorbic acid) and lycopene.

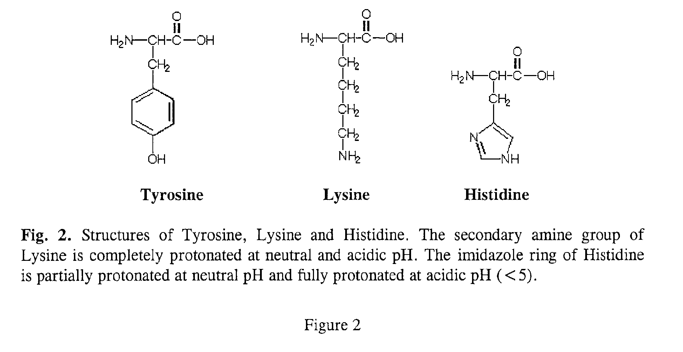

For a radical scavenger to be effective in protecting a protein, the scavenger molecules should be physically close to and/or associated with the amino acids most susceptible to free radical attack. This means that to have a protective effect, a compound should be attracted (e.g. by hydrophobic interactions, electrostatic interactions etc.) to the side chains of the 8 vulnerable amino acids identified by our analysis, namely leucine, isoleucine, phenylalanine, tryptophan, methionine, histidine, tyrosine and lysine. The side chains of leucine, isoleucine, phenylalanine, tryptophan and methionine are uncharged and very non-polar. They are therefore capable of engaging in hydrophobic interactions with similar non-polar molecules. The structures of the remaining three amino acids, histidine, tyrosine and lysine, are shown in Fig. 2. Two of them, histidine and lysine, carry a positive charge on their side chains at near-neutral or acidic pH. In the case of lysine the secondary amino group is fully protonated at neutral and acidic pH (pK = 8.9), and in the case of histidine the imidazole ring is partially protonated at neutral pH and fully protonated at acidic pH (< 5) (pK = 6.0). These side chains can therefore engage in electrostatic interactions with negatively charged molecules. Although all three amino acids are hydrophilic, either due to their positive charge (histidine and lysine) or the hydroxyl group (tyrosine), a considerable part of their molecular structure is capable of engaging in hydrophobic interactions (i.e. via the benzene ring of tyrosine, the alkyl chain of four methylene groups of lysine and the imidazole ring of histidine).

It can be concluded that the amino acids most likely to be targeted by the hydroxyl radical and hydrated electron can engage in electrostatic interactions with negatively charged molecules (in the case of histidine, lysine and tyrosine) and/or in hydrophobic interactions.

Based on this analysis, it is possible to select a chemical compound or combination of chemical compounds that will function to protect a protein in an aqueous environment against loss of activity on exposure to ionising radiation.

### 4. Summary of the invention

In one aspect, the invention provides a method of sterilising a protein in an aqueous environment, comprising (a) bringing the protein into contact with a protective compound or combination of protective compounds having the following characteristics:
(i) a rate of reaction with hydroxyl radicals greater than 5 x 10⁸ L mol⁻¹ s⁻¹; and
(ii) a non-polar region,
   excluding the case where the protein is an enzyme and the protective compound(s) comprise a source of lactate ions and a source of zinc ions and/or a source of ammonium ions and excluding guanine and histidine as protective compounds; and (b) exposing the protein and protective compound(s) to ionising radiation.

In this specification, all references to values for rate of reaction , or rate values, are at 25°C unless otherwise specified.
The protection may be complete, i.e. with 100% retention of activity, so that no activity is lost on exposure to ionising radiation, or may be partial, with less than 100 % retention of activity, so that some (but not all) activity is lost on exposure to ionising radiation. In most practical cases only partial protection is achieved, but this still provides useful benefits, as without use of the protective compound or compounds all activity will generally be lost on exposure to ionising radiation sufficient to achieve sterilisation. The retention of activity is preferably at least 5 % , more preferably at least 10 %, 20 %, 30 % , 40%, 50%, 60%, 70%, 80% or 90%.

The ionising radiation is typically in the form of gamma radiation, electron beam radiation or X-ray radiation.

In a further aspect, the invention provides a method of stabilising a protein in an aqueous environment during sterilisation by exposure to ionising radiation, comprising bringing the protein into contact with a protective compound or combination of protective compounds having the following characteristics:
(i) a rate of reaction with hydroxyl radicals greater than 5 x 10⁸ L mol⁻¹ s⁻¹; and
(ii) a non-polar region,
   excluding the case where the protein is an enzyme and the protective compound(s) comprise a source of lactate ions and a source of zinc ions and/or a source of ammonium ions and excluding guanine and histidine as protective compounds

The invention also provides a composition comprising a protein in an aqueous environment and a protective compound or combination of protective compounds having the following characteristics:
(i) a rate of reaction with hydroxyl radicals greater than 5 x 10⁸ L mol⁻¹ s⁻¹; and
(ii) a non-polar region,
   excluding the case where the protein is an enzyme and the protective compound(s) comprise a source of lactate ions and a source of zinc ions and/or a source of ammonium ions, excluding the case where the protein is bilirubin oxidase and the protective compound is tryptophan, excluding the case where the enzyme is lactate dehydrogenase and the protective compound is malic acid salt, excluding the case where the enzyme is lactate dehydrogenase and the protective compound is succinic acid salt, and excluding guanine and histidine as protective compounds

The composition has desirably been sterilised by exposure to ionising radiation. The invention covers a protein in microbiologically sterile condition, after exposure to ionising radiation.

The required characteristics, namely a rate of reaction with hydroxyl radicals greater than 5 x 10⁸ L mol⁻¹ s⁻¹ and the non-polar region, may both be present in a single protective compound or may be separately present in two different compounds (one compound satisfying the reaction rate requirement and the other compound having a non-polar region) that together form a combination of protective compounds. It is also possible for individual members of a combination of protective compounds to satisfy both requirements.

Because the protective compound or combination of protective compounds satisfies the requirement of having a comparatively high rate of reaction with hydroxyl radicals, greater than 5 x 10⁸ L mol⁻¹ s⁻¹, preferably greater than 10⁹ L mol⁻¹ s⁻¹, the compound(s) is likely to react with hydroxyl radicals generated from water associated with the protein on exposure to ionising radiation in preference to the hydroxyl radicals reacting with the protein and causing degradation and loss of activity. Further, because the protective compound or combination of protective compounds satisfies the requirement of having a non-polar (hydrophobic) region, it will be attracted to the vulnerable protein side chains, leucine, isoleucine, phenylalanine, tryptophan, methionine, histidine, tyrosine and lysine, as explained above, and will be able to engage in desirable hydrophobic interactions therewith. These two effects mean that the protective compound(s) function to protect the protein from degradation and loss of activity on exposure to ionising radiation.The protective compound or combination of protective compounds preferably satisfy the requirement of having a non-polar region that does not have a positive charge directly on the non-polar region, as the presence of a positive charge can in some cases compromise the protective effect by limiting the ability of the non-polar region to engage in desirable hydrophobic interactions with the vulnerable protein side chains. Where a compound includes more than one polar region it is possible for one (or more) of these to have a positive charge directly thereon provided there is at least one non-polar region without a positive charge. For example, thiamine contains two detached non-polar rings, one of which has a positive charge and the other of which is charge-free, and functions well as a protective compound.

One or more compounds of the protective compound or combination of protective compounds preferably also satisfy the requirement of having a negative charge at neutral pH, for added attraction to histidine and lysine (which are positively charged at neutral and acidic pH).

One or more compounds of the protective compound or combination of protective compounds preferably also satisfy the requirement of having a comparatively high rate of reaction with hydrated electrons, greater than 10⁸ L mol⁻¹ s⁻¹, preferably greater than 5 x 10⁸ L mol⁻¹ s⁻¹, more preferably greater than 10⁹ L mol⁻¹ s⁻¹, for additional protective effect.

The non-polar region is typically an aliphatic chain, heterocyclic or aromatic ring structure that is capable of forming non-covalent hydrophobic bonds with the side chains of hydrophobic amino acids such as tryptophan, lysine or histidine. In particular, the non-polar region can consist of an aliphatic chain (ie a chain consisting of combinations of carbon and hydrogen atoms) comprising at least two carbon atoms, preferably at least three carbon atoms and most preferably more than three carbon atoms. The chain can include single, double and triple bonds. Carbon can be substituted in the chain by an atom of comparable electronegativity, typically sulphur or nitrogen. The chain can be linear or branched.

The non-polar region can also consist of a cycle of at least four carbon atoms accompanied by an appropriate number of hydrogen atoms. The cycle can include single and/or double bonds. Carbon can be substituted in the cycle by an atom of comparable electronegativity, typically sulphur or nitrogen.

The non-polar region can also consist of an aromatic structure, ie a structure including at least one benzene nucleus.

Preferably, one or more of the protective compound or compounds possesses a non-polar region to which one or a limited number of polar groups (e.g. hydroxyl) is attached. This improves the solubility of the compound(s) in water.

Examples of a suitable single protective compound or combinations of protective compounds identified using these criteria are given below.

### Single compound

The compound must be capable of rapid reaction with the dominant free radicals produced in aqueous systems on gamma-irradiation, particularly with hydroxyl radicals.The compound will have a high rate of reaction with both hydroxyl radicals (greater than 5 x 10⁸ L mol⁻¹ s⁻¹, preferably greater than 10⁹ L mol⁻¹ s⁻¹) and preferably also has a high rate of reaction with hydrated electrons (greater than 10⁸ L mol⁻¹ s⁻¹, preferably greater than 5 x 10⁸ L mol⁻¹ s⁻¹, more preferably greater than 10⁹ L mol⁻¹ s⁻¹).

In addition, the compound must possess a non-polar (hydrophobic) region (e.g. an aliphatic chain, heterocyclic or aromatic ring). The compound preferably also possesses a negative charge at neutral pH.

The compound can be completely non-polar (i.e. uncharged and in the absence of polar groups such as hydroxyl- or amino-). An example of such a compound is 1,10-phenanthroline. Although effective in protecting the protein, a disadvantage of such compounds is their poor solubility in water.

Preferably, therefore, the compound possesses a non-polar region to which one or a limited number of polar groups (e.g. hydroxyl) is attached. This improves the solubility of the compound. An example of such a compound is methoxyphenol.

Most preferably, the compound possesses a non-polar region to which a negative charge is attached. Examples of such compounds are tryptophan and nicotinic acid.

If a negative charge is present in the molecule at neutral/physiological pH, then the presence of positive charge is not necessarily detrimental, especially if it is not positioned in direct contact with the non-polar region, for instance as in tryptophan or adenine.

A positive charge directly adjacent to the non-polar part of the molecule (e.g. on the heterocyclic ring) is likely to be detrimental, as in histidine or guanine, unless the compound also includes another non-polar region without positive charge, as with thiamine.

### Combination of compounds

The compounds must be capable of rapid reaction with the dominant free radicals produced in aqueous systems on gamma-irradiation, particularly hydroxyl radicals. Thus, at least one of the compounds must have a high rate of reaction with hydroxyl radicals (greater than 5 x 10⁸ L mol⁻¹ s⁻¹, preferably greater than 10⁹ L mol⁻¹ s⁻¹). Preferably, at least one of the compounds has a high rate of reaction with hydroxyl radicals (greater than 5 x 10⁸ L mol⁻¹ s⁻¹, preferably greater than 10⁹ L mol⁻¹ s⁻¹) and at least one of the compounds has a high rate of reaction with hydrated electrons (greater than 10⁸ L mol⁻¹ s⁻¹, preferably greater than 5 x 10⁸ L mol⁻¹ s⁻¹, more preferably greater than 10⁹ L mol⁻¹ s⁻¹).

In addition, at least one of the compounds should possess a non-polar (hydrophobic) region (e.g. an aliphatic chain, heterocyclic or aromatic ring). Preferably at least one of the compounds possessesa negative charge at neutral pH.

Both or all compounds can be completely non-polar (i.e. uncharged and in the absence of polar groups such as hydroxyl- or amino-). Although effective in protecting the protein the disadvantage of such compounds is their poor solubility in water.
Preferably, therefore, at least one compound possesses a non-polar region and at least one compound possesses a polar group, such as an hydroxyl group. An example of such a combination is methoxyphenol and mannitol.

Most preferably, at least one compound possesses a non-polar region and at least one compound possesses a negative charge. An example of such a combination is methoxyphenol and iodide.

Direct competition of negative charges is likely to be detrimental and should be avoided, for instance the combination of iodide and nitrate.

However, negative charges on more than one compound might be allowed if at least one molecule is large and direct competition of anions can thus be avoided due to the steric effects. An example of such a combination is nitrate and tryptophan.

If a negative charge is present, then positive charge is not necessarily detrimental, especially if it is not positioned in direct contact with the non-polar region.

A positive charge positioned directly on the non-polar part of one of the compounds (e.g. on the heterocyclic ring) is likely to be detrimental, as in the combination of histidine and guanine.

Examples of compounds that fit the criteria for protecting proteins through gamma irradiation are listed below. However, this is only a limited selection of compounds and the invention is not at all limited by these. Some general trends that can be used to identify a suitable compound or combination of compounds are also explained.

(N.B. The reaction rates of compounds that are not listed in the www.rcdc.nd.edu internet source are referred to as "not known")

### A. Aromatic compounds

Practically every aromatic organic compound (i.e. compound containing at least one benzene nucleus) has a good rate (> 1 × 10⁹) of reaction with hydroxyl radical. Many of these also have a good rate of reaction (> 1 × 10⁹) with the hydrated electron. Due to the presence of the benzene nucleus, all aromatic compounds have a substantial non-polar region, which makes them good candidates for the protection of proteins through gamma irradiation. It is beneficial if the aromatic compounds contain the negative charge (to increase their solubility and improve their interaction with the target amino acids) or a hydrophilic group (to increase their solubility). Examples of the suitable aromatic compounds are shown in Table below:

| **Compound** | **k(OH·) / L mol⁻¹ s⁻¹** | **k(e⁻_{aq}) / L mol⁻¹ s⁻¹** |
|---|---|---|
| | | |

| **Completely non-polar** | | |
|---|---|---|
| 1,10-Phenanthroline | 7.0 × 10⁹ | 1. 8 × 10¹⁰ |
| Benzene | 1.1 × 10¹⁰ | 9.0 × 10⁶ (insufficient) |
| Benzaldehyde | 4.4 × 10⁹ | 2.4 × 10¹⁰ |
| Biphenyl | 1.0 × 10¹⁰ | 1.2 × 10¹⁰ |
| Cumene | 7.5 × 10⁹ | 4.4 × 10⁹ |
| Indole | 3.2 × 10¹⁰ | 2.6 × 10⁸(insufficient) |
| Naphthalene | 9.4 × 10⁹ | 5.0 × 10⁹ |
| *o*-Xylene | 6.7 × 10⁹ | Not known |
| 1,4-Benzoquinone | 1.2 × 10⁹ | 2.3 × 10¹⁰ |
| | | |

| **With polar groups** | | |
|---|---|---|
| Phenol | 6.6 × 10⁹ | 3.0 × 10⁷ (insufficient) |
| Benzidine | 1.5 × 10¹⁰ | 2.1 × 10⁹ |
| 1,2-Benzenediol | 1.1 × 10¹⁰ | Not known |
| Nicotinamide | 1.4 × 10⁹ | 2.4 × 10¹⁰ |
| 1-Phenylethanol | 1.1 × 10'° | Not known |
| 1-Phenyl-2-propanol | 2.1 × 10¹⁰ | Not known |
| 5-Hydroxyindole | 1.7 × 10¹⁰ | Not known |
| Phenylthiourea | 3.8 × 10⁹ | 4.2 × 10⁹ |
| Methoxyphenol | 2 × 10¹⁰ | 9.7 × 10⁹ |
| | | |

| **With polar groups and/or** negative charge | | |
|---|---|---|
| Benzoate ion | 5.9 × 10⁹ | 3.0 × 10⁹ |
| Cinnamate ion | 8.1 × 10⁹ | 1.4 × 10¹⁰ |
| Folic acid | 9.6 × 10⁹ | 2.2 × 10¹⁰ |
| Nicotinate ion | 2.5 × 10⁹ | 6 × 10⁹ |
| Phenylalanine | 6.5 × 10⁹ | 1.2 × 10⁸(insufficient) |
| Salicylate ion | 1.2 × 10¹⁰ | 1 × 10¹⁰ |
| Tryptophan | 1.3 × 10¹⁰ | 1.4 × 10¹⁰ |
| Tyramine anion | 1.5 × 10¹⁰ | 5.8 × 10⁷ (insufficient) |
| Tyrosine | 1.3 × 10¹⁰ | 2.8 × 10⁸ (insufficient) |
| Aminobenzoate | 1.1 × 10¹⁰ | 1.9 × 10⁹ |

### B. Heterocyclic compounds

Most heterocyclic compounds (i.e. compounds where one or more carbon atoms in a cycle is replaced by nitrogen, oxygen or sulphur) have a good rate (> 1 × 10⁹) of reaction with hydroxyl radical. Some of these also have good rate of reaction (> 1 × 10⁹) with the hydrated electron. Due to the presence of the carbon/heteroatom cycle, the heterocyclic compounds have a substantial non-polar region, which makes them good candidates form the protection of proteins through gamma irradiation. It is beneficial if the heterocyclic compounds contain the negative charge (to increase their solubility and improve their interaction with the target amino acids) or a hydrophilic group (to increase their solubility). Examples of the suitable heterocyclic compounds are shown in Table below:

| **Compound** | **k(OH•) / L mol⁻¹ s⁻¹** | **k(e⁻_{aq}) / L mol⁻¹ s**^{**-**1} |
|---|---|---|
| Uric acid | 7.2 × 10⁹ | 6 × 10⁹ |
| Uridine | 5.2 × 10⁹ | 1.4 × 10¹⁰ |
| Uracil | 5.7 × 10⁹ | 1.5 × 10¹⁰ |
| Thymine | 6.4 × 10⁹ | 1.8 × 10¹⁰ |
| Thiamine | 3.0×10⁹ | 3.4×10¹⁰ |
| Riboflavine | 1.2 × 10¹⁰ | 2.3 × 10¹⁰ |
| Pyridoxine | 6.3 × 10⁹ | 2.2 × 10¹⁰ |
| Pyridine | 3.0 × 10⁹ | 7.7 × 10⁹ |
| Phthalate ion | 5.9 × 10⁹ | 4.6 × 10⁹ |
| Imidazole | 5.2 × 10⁹ | 2.0 × 10⁷ (insufficient) |
| Orotate ion | 6.5 × 10⁹ | 1.4 × 10¹⁰ |
| Flavine mononucleotide | 2.5 × 10⁹ | Not known |
| Cytidine | 5.8 × 10⁹ | 1.3 × 10¹⁰ |
| Cytosine | 6.3 × 10⁹ | 1.3 × 10¹⁰ |
| Caffeine | 6.9 × 10⁹ | 1.2 × 10¹⁰ |
| 3-Pyridinol | 8.9 × 10⁹ | 1.4 × 10¹⁰ |
| Adenine | 5.8 × 10⁹ | 9.0 × 10⁹ |
| Adenosine | 5.8 × 10⁹ | 1.0 × 10¹⁰ |
| Bilirubin dianion | 1.3 × 10¹⁰ | 1.7 × 10¹⁰ |

### C. Compounds containing one or more hydroxyl groups

Most compounds containing a hydroxyl group (preferably more than one) tend to have a good rate of reaction (> 1 × 10⁹) with hydroxyl radical. In contrast, the presence of hydroxyl group does not at all guarantee a good rate of reaction with hydrated electron, so to achieve the best results the hydroxyl compounds should be used in mixture with compounds that are reactive with hydrated electron. Examples of the hydroxyl compounds with substantial non-polar region that satisfy the requirements for the effective protection of proteins through gamma irradiation are shown in the Table below

| **Compound** | **k(OH·) / L mol⁻¹ s⁻¹** | **k(e⁻_{aq}) / L mol⁻¹ s⁻¹** |
|---|---|---|
| 1-Propanol | 2.8 × 10⁹ | Not known |
| 1-Pentanol | 3.7 × 10⁹ | Not known |
| 1-Naphthol | 1.3 × 10¹⁰ | Not known |
| 2-Methoxyethanol | 1.3 × 10⁹ | Not known |
| 1-Heptanol | 7.4 × 10⁹ | Not known |
| 1-Hexanol | 7.0 × 10⁹ | Not known |
| 2,2-Dimethyl-1-propanol | 5.2 × 10⁹ | Not known |
| Butanediol | 2.2 × 10⁹ | 8 × 10⁹ |
| 1-Butanol | 3.1 × 10⁹ | Not known |
| Allyl alcohol | 5.9 × 10⁹ | Not known |

### D. Organic acids

Some organic acids have a good rate of reaction (> 1 × 10⁹) with hydroxyl radical. Some of those also have a good rate of reaction with hydrated electron. Examples of organic acids with substantial non-polar region that satisfy the requirements for the effective protection of proteins through gamma irradiation are shown in the Table below. Each acid will exist as a mixture of the dissociated form (i.e. as an anion) and the protonated form. The pKa of the acid and pH of the environment determines which form will predominate. In same cases (e.g. lactate/lactic acid) both forms are needed to provide protection of the protein both with respect to hydroxyl radical and hydrated electron.

| **Compound** | **k(OH·) / L mol⁻¹ s⁻¹** | **k(e⁻_{aq}) / L mol⁻¹s⁻¹** |
|---|---|---|
| | | |
| Lactic acid/Lactate: | | |
| Lactate ion | 1.6 × 10¹⁰ | 1 × 10⁷ (insufficient) |
| Lactic acid | 4.3 × 10⁸ (insufficient) | 7.0 × 10⁹ |
| Maleic acid/Maleate: | | |
| Maleate ion | Not known | 1.7 × 10⁹ |
| Maleic acid | 6.0 × 10⁹ | 2.9 × 10¹⁰ |
| Malic acid/Malate: | | |
| Malate ion | 2.3 × 10⁹ | 6 × 10⁷ (insufficient) |
| Malic acid | 8.2 × 10⁸ (insufficient) | 3 × 10⁹ |
| Acrylic acid/Acrylate: | | |
| Acrylic acid | 1.5 × 10⁹ | 2.4 × 10¹⁰ |
| Acrylate ion | 5.6 × 10⁹ | 5.3 × 10⁹ |
| Sulphanilic acid/Sulphanilate | | |
| Sulphanilic acid | Not known | 5.9 × 10⁹ |
| Sulphanilate | 8.2 × 10⁹ | 4.6 × 10⁸ (insufficient) |
| Fumaric acid/Fumarate | | |
| Fumaric acid | 6.0 × 10⁹ | Not known |
| Fumarate ion | Not known | 7.4 × 10⁹ |
| Adipic acid | 2.0 × 10⁹ | Not known |
| Methacrylate ion | 2.1 × 10¹⁰ | 4.5 × 10⁹ |
| Butyrate ion | 2 × 10⁹ | Not known |

### E. Oxidisable ions

Various oxidisable inorganic ions have a good rate of reaction (> 1 × 10⁹) with hydroxyl radical. These typically do not have a good rate of reaction with hydrated electron. Many of the inorganic ions lack the hydrophobic region, so they need to be used in conjunction with other compounds that can provide the hydrophobic interaction. Examples of some of the ions are shown in table below:

| **Compound** | **k(OH·) / L mol⁻¹ s⁻¹** | **k(e⁻_{aq}) / L mol⁻¹ s⁻¹** |
|---|---|---|
| | | |
| Bromide ion | 1.1 × 10¹⁰ | Not known |
| Chloride ion | 3.0×10⁹ | 1×10⁶ (insufficient) |
| Ferrocyanide ion | 1.1 × 10¹⁰ | 7 × 10⁴ (insufficient) |
| Iodide ion | 1.1 × 10¹⁰ | 2.4 × 10⁵ (insufficient) |
| Hydroxide ion | 1.3×10¹⁰ | Not known |
| Sulphite ion | 5.1 × 10⁹ | 1.5 × 10⁶ (insufficient) |
| Bisulphide ion | 9.0 × 10⁹ | 3.0 × 10⁷ (insufficient) |
| Thiosulphate ion | 7.8×10⁹ | 1.0×10⁸ (insufficient) |
| Bromite ion | 2.3 × 10⁹ | 1.8 ×10¹⁰ |
| Chlorite ion | 7 × 10⁹ | 2.5 × 10⁹ |

### F. Other suitable compounds

Other examples of compounds that fit the structural requirements and have a good rate of reaction with hydrated electron and/or hydroxyl radical are listed in the table below.

| **Compound** | **k(OH·) / L mol⁻¹ s⁻¹** | **k(e⁻_{aq}) / L mol⁻¹ s⁻¹** |
|---|---|---|
| | | |
| Diethyl ether | 2.9 × 10⁹ | 1 × 10⁷ (insufficient) |
| Diethyl sulphoxide | 6.5 × 10⁹ | Not known |
| Dimethyl sulphide | 1.9×10¹⁰ | Not known |
| Dipropyl sulphoxide | 6.3 × 10⁹ | Not known |
| Diethylene glycol diethyl ether | 3.2 × 10⁹ | Not known |
| Camphor | 4.1 × 10⁹ | 3.1 × 10⁹ |
| Crotonaldehyde | 5.8 × 10⁹ | Not known |
| Acetaldehyde | 3.6 × 10⁹ | 4.4 × 10⁹ |
| Acrylamide | 5.9 × 10⁹ | 1.5 × 10¹⁰ |
| Cysteine | 1.9 × 10¹⁰ | 8.5 × 10⁹ |
| Cystine | 2.1 × 10⁹ | 1.1 × 10¹⁰ |
| Acrolein | 7.0 × 10⁹ | Not known |

### G. Compounds with a good rate of reaction with hydrated electron

Compounds with a good rate of reaction with hydrated electron that either contain a non-polar region or are negatively charged cannot protect protein through gamma irradiation. However, they can be still useful in conjunction with other compounds that react effectively with hydroxyl radical. Examples of such compounds are shown in table below.

| **Compound** | **k(OH·) / L mol⁻¹ s⁻¹** | **k(e⁻_{aq}) / L mol⁻¹ s⁻¹** |
|---|---|---|
| | | |
| Nitrated organic compounds | Various | Typically higher than 10⁹ |
| Nitrite ion | 6.0×10⁶ (insufficient) | 3.5×10⁹ |
| Nitrate ion | Not known | 9.7 × 10⁹ |
| Pyruvate ion | 3.1 × 10⁷(insufficient) | 6.8×10⁹ |
| Pyrimidine | 1.6 × 10⁸(insufficient) | 2.0 × 10¹⁰ |
| Pteridine | Not known | 3.0 × 10¹⁰ |
| Pterin | Not known | 2.5 × 10¹⁰ |
| Picrate ion | Not known | 3.9 × 10¹⁰ |
| Benzyl acetate | Not known | 1.1 × 10⁹ |
| Benzoin | Not known | 1.7 × 10¹⁰ |
| *N*-Acetylcysteamine | Not known | 9.1 × 10⁹ |
| Acetone | 1.3 × 10⁸(insufficient) | 7.7 × 10⁹ |
| Iodate ion | < 10⁵ | 5.4 × 10⁹ |
| Ferricyanide ion | Not known | 3.1 × 10⁹ |
| Coenzyme B₁₂ | Not known | 3.2 × 10¹⁰ |
| Bromate ion | 5 × 10⁶ | 3.4 × 10⁹ |

For any particular protein, selection of appropriate protective compound(s) will include consideration of criteria including solubility, toxicity, process considerations, undesirable physical or chemical protein interactions, etc.

The protective compound(s) may optionally be used in combination with other ingredients known to stabilise enzymes (hereinafter for brevity and simplicity referred to as "stabilisers") .

Suitable known stabilisers for use herein include sugar alcohols such as mannitol, sorbitol, xylitol and lactitol; proteins such as gelatin; and neutral water-soluble polymers such as polyvinyl pyrrolidone and polyvinyl alcohol (e.g. having a molecular weight in the range of about 30,000 to 100,000).

Sugar alcohols can typically be used as stabilisers at concentrations in the range 0.5% to 4% by weight.

If proteins are employed as stabilisers, they may be present at a concentration of at least 0.5 % , preferably at least 1 %, and more preferably at least 4 %, by weight.

Neutral water-soluble polymers can be used with good effect as stabilisers, typically at concentrations in the range of 0.5 % to 3.5 % by weight.

The invention is applicable to a wide range of proteins, with protection of enzymes being of particular practical importance.

The term "protein" is used herein to encompass molecules or molecular complexes consisting of a single polypeptide, molecules or molecular complexes comprising two or more polypeptides and molecules or molecular complexes comprising one or more polypeptides together with one or more non-polypeptide moieties such as prosthetic groups, cofactors etc. The term "polypeptide" is intended to encompass polypeptides comprising covalently linked non-amino acid moieties such as glycosylated polypeptides, lipoproteins etc. In particular the invention relates to molecules having one or more biological activities of interest which activity or activities are critically dependent on retention of a particular or native three dimensional structure in at least a critical portion of the molecule or molecular complex. In general it is thought the invention is applicable to polypeptides with a molecular weight of at least 2000 (i.e. consisting of at least about 15 amino acids) where at least basic motifs of secondary or tertiary structure possibly important for protein function might be formed.

In general, especially with proteins for medical use, it will be desirable to use the compound(s) in as low a concentration as possible while still obtaining effective protection. The protective compound(s) are typically used at a concentration in the range 1mM to 1M, preferably 1mM to 200mM, most preferably 5mM to 100mM.

The invention is further described, by way of illustration, in the following Examples and

with reference to the accompanying Figures, in which:
Figures 1A, 1B and 1C are graphs of rate constant k (in L mol⁻¹ s⁻¹) for L-amino acids with hydroxyl radical (shown by bold bars) and with hydrated electron (shown by faint bars); and
Figure 2 shows the structures of the amino acids tyrosine, lysine and histidine.

### 5. Examples

### Materials & methods

### Chemicals & other materials

Water (conductivity < 10 *µ*S cm⁻¹; either analytical reagent grade, Fisher or Sanyo Fistreem MultiPure)
Glucose Oxidase - Biocatalysts - G638P (~70 U /mg solid)
Lactoperoxidase (from bovine milk, DMV International: 1,050 units mg⁻¹ by ABTS method pH 5.0)
Catalase (from bovine liver, Sigma C9322, 2380 U /mg solid)
Glucose - Fisher - analytical grade, code G050061
All compounds tested as protecting agents were of analytical grade

### Overall Experimental plan

In each example, an aqueous solution of a protein was prepared with selected additives in an Ependorf tube. The Eppendorf tubes were delivered to and gamma-irradiated by an industrial sterilisation service, with a dose range typical for sterile medical products. The gamma-irradiated solutions were returned and analysed for protein activity.

### Gamma irradiation

The samples (approx. 1.5 ml in a 2 ml Ependorf tube) were gamma-irradiated by an industry-standard commercial sterilising service provided by Isotron PLC (Swindon, Wilts, UK), using a Cobalt 60 gamma source. The radiation dose was in the range of 25 - 40 kGy.

### Glucose oxidase activity assay.

The solutions contained 100 µg mL⁻¹ of glucose oxidase. The solutions, both pre- and post-gamma irradiated, were assayed for glucose oxidase activity. This was performed according to the following procedure:
50 µL of the solution was added to 50 mL of deionised water. The following solutions were then added:
- 10 mL of reagent mix (5 parts of 0.1 M sodium phosphate, pH 6 + 4 parts 2 % w/w starch + 1 part of 1mg/mL lactoperoxidase enzyme);
- 5 mL of 100 mM potassium iodide and
- 5 mL of 20% w/w glucose solution.

These were mixed together quickly. Time = 0 was counted from the addition of the glucose. After 5 min, 1 ml of 5 M aq. hydrochloric acid was added to stop the reaction. The absorbance was then read at 630 nm using a Unicam UV-visible spectrophotometer (Type: Helios gamma). If the colour intensity was too great to allow an accurate reading, the sample was diluted with a defined volume of deionised water to bring the colour back on scale. The results were expressed as percentage recovery, by reference to the absorbance measured in the pre-gamma irradiation samples.

### Catalase activity assay

The solutions contained 100 µg mL⁻¹ of catalase. The solutions, both fresh and after incubation at increased temperature, were assayed for catalase activity. This was performed according to the following procedure:

2 mL of hydrogen peroxide (30 mM in water) was added to 18 mL of PBS in a 125 mL polypropylene pot. 100 µL of the catalase sample was added and mixed. The resulting mixture was incubated at room temperature precisely for 30 min. In the meantime, the following reagents were mixed in a plastic cuvette for spectrophotometric measurements:
- 2.73 mL of citrate/phosphate buffer (0.1 M, pH 5.0)
- 100 *µ*L of tetramethylbenzidine (TMB) (3 mg/mL, dissolved in dimethyl sulphoxide (DMSO))
- 100 *µ*L of lactoperoxidase

Following the 30 min incubation period, 70 *µ*L of the catalase containing mixture was added to the cuvette and absorbance was read in approximately 30 s. The results were expressed as percentage recovery, by reference to the absorbance measured in the fresh samples (i.e. prior to incubation at increased temperature).

### Monoclonal Antibody ELISA Method:

Nunc Maxisorp F96 (Fisher, code DIS-971-030J) multi-well plates were sensitised with 100*µ*l per well of a 2.5*µ*g/ml hCG (Sigma, code C5297) in 0.1M sodium carbonate pH 8.4 (Fisher, code S/4240/53) solution. The plates were incubated at 4°C, for 18hours. After sensitisation, the hCG solution was removed, and the plates washed 5x with Tris-buffered Saline Tween (TBST) (20mM Tris (Fisher, code BPE152-1) + 137mM NaCl (Fisher, code S/3160/63) + 0.05%v/v Tween 20 (Fisher, code BPE337-100), pH 7.6)). Blocking was carried out with 0.5mg/ml bovine serum albumin (BSA) (Sigma, codeA7030) in 0.1M sodium carbonate pH 8.4 for 1hour at 37°C followed by washing 5x in TBST. After washing, the test (primary) antibody was added at 5*µ*g/ml in TBST, at 100*µ*l per well. The plates were incubated for 2hours at 37°C, then washed 5x with TBST. Following this, 100*µ*l per well of an anti-mouse - horseradish peroxidase (HRP) conjugate (Sigma, code A8924) was added, at 1/2500 dilution in TBST (secondary antibody). This was then incubated for 1hour at 37°C, before washing 5x with TBST. Development was carried out using ready prepared 2,2'-azino-bis(3-ethylbenzothiazoline-6-sulphonic acid) (ABTS) (Sigma, code A3219), 100*µ*l per well. Colour development was monitored on a Dynatech MR5000 plate reader, at 405nm. Two control wells were included, with either the primary or secondary antibodies omitted from the assay. This would therefore check for any nonspecific binding and determine background reagent interference.

### Papain assay

The papain solutions, both fresh and after incubation at increased temperature, were assayed for papain activity. This was performed according to the following procedure:

100 µl of the papain sample was mixed with 100 µL of cysteine (24 mg/mL prepared in 25 mM phosphate buffer, pH 6.9). 160 µL of ethylenediaminetetraacetic acid (EDTA) (2.5 mM prepared in 250 mM phosphate buffer, pH 6.0) was added and the resulting mixture was incubated at 60 °C for 10 min. 160 *µ*L of N α-benzoyl-DL-arginine-β-naphthylamide hydrochloride (BANA) (5 mg/mL prepared in 20% DMS0180 % water) was added and incubated at 60°C for another 10 min. The reaction was stopped by addition of 280 *µ*l of HCl/methanol mixture (prepared by mixing 1 mL of 5 M HCl and 9 mL of methanol). 400 *µ*L of 4-(dimethylamino) cinnamaldehyde (DMAC) was added and the final mixture was allowed to stand at room temperature for 25 min. Absorbance of the mixture was then measured at 540 nm. If the colour intensity was too great to allow an accurate reading, the sample was diluted with a defined volume of 80% (v/v) methanol (prepared by mixing 4 volume parts of methanol and 1 volume part of deionised water) to bring the colour back on scale. The results were expressed as percentage recovery, by reference to the absorbance measured in the fresh samples (i.e. prior to incubation at increased temperature).

The following fifteen sets of examples summarise the results of our practical investigations into the protective effect of various potential protective compounds (singly or in combination) on the recover of measurable protein activity after gamma sterilisation of aqueous solutions. Most examples presented here (example 1 - example 11) were acquired using the primary model enzyme - glucose oxidase. Nevertheless, some examples are also presented using other model enzymes or monoclonal antibodies. In all of the examples, the specified protective compound(s) at the specified concentration were mixed with an aqueous solution of the protein under test, the solutions were irradiated with gamma radiation, and the activity of the protein was tested as specified. In the results the rates of reaction, k, are expressed as L mol⁻¹s⁻¹.

### Example 1: Single compounds that did not protect aqueous glucose oxidase against the effects of gamma radiation due to their insufficient rates of reaction with the hydroxyl radical.

Under the conditions described, a rate of reaction with hydroxyl radicals greater than about 5 x 10⁸ L mol⁻¹ s⁻¹ is an essential pre-requisite of the protein protecting effect. Table 1 shows a selection of single compounds that did not protect glucose oxidase against the effects of gamma irradiation because of their insufficient rates of reaction with hydroxyl radicals and hydrated electrons. The hydroxyl radical rate constant of nitrate was not listed, but is thought to be low. The hydrated electron rate constant of nitrate is sufficiently high, but did not compensate for the poor hydroxyl radical reaction rate.

**Table 1.**

| **Compound** | **Concentration** | **Rate of reaction with hydroxyl radicals k(OH·)** | **Rate of reaction with hydrated electrons k(e⁻ _{aq})** | **Enzyme activity remaining after gamma-irradiation** |
|---|---|---|---|---|
| | | | | |
| Nitrate | 20 mM | N/A | 9.7 × 10⁹ | 0 % |
| Proline | 20 mM | 3.1×10⁸ | 2 × 10⁷ | 0 % |
| Glycine | 20 mM | 1.7× 10⁷ | 1 × 10⁷ | 0 % |

### Example 2: Single compounds, with high rates of reaction with hydroxyl radicals, that did not confer enzyme protection.

The sufficient rate of reaction with hydroxyl radical (arbitrarily selected as > 5 x 10⁸ L mol⁻¹ s⁻¹) is an essential pre-requisite of the protecting effect. This condition is met in the case of the two compounds shown in Table 2 (in fact, iodide is often referred to as hydroxyl radical scavenger). Nevertheless, these compounds did not offer any protection of glucose oxidase for the following reasons. Firstly, their reaction rates with hydrated electron are extremely low, and secondly (and more importantly), there are no non-polar regions in those two molecules that would allow effective interaction with histidine, lysine and tyrosine.

**Table 2.**

| **Compound** | **Concentration** | **k(OH·)** | **k(e⁻_{aq})** | **Activity Retention** |
|---|---|---|---|---|
| | | | | |
| Iodide | 20 mM | 1×10¹⁰ | 2.4×10⁵ | 0 % |
| Mannitol | 20 mM | 2.5×10⁹ | 7×10⁶ | 0 % |

### Example 3: Compounds with high rates of reaction with both hydroxyl radicals and hydrated electrons whose effectiveness to protect glucose oxidase was compromised by a positive charge on the non-polar ring.

The compounds listed in Table 3 have very high rates of reaction with both hydroxyl radicals and hydrated electrons. Their molecules also have extensive non-polar regions capable of engaging in hydrophobic interactions. This should make them good protecting agents for the aqueous enzyme against the effects of gamma irradiation. Nevertheless their ability to protect the enzyme is compromised by the presence of positive charge (at neutral and acidic pH) directly on the heterocyclic rings of their molecules. In the case of guanine and histidine the positive charge resulted in no protective effect. In the case of uracil some protective effect was observed. Nevertheless, the protective effect of uracil was smaller than that of cytosine (see Table 5), a very similar compound which lacked a positive charge on its heterocycle. The fact that two of the compounds presented in Table 3 did not offer any protection while one offered some protection indicates that the rule asserting the presence of positive charge as detrimental to the protecting effect can only be seen as an indication of a general trend rather than a strict rule. The final effect can be further affected by various steric effects and will thus depend on the particular molecules.

**Table 3.**

| **Compound** | **Concentration** | **k(OH·)** | **k(e⁻_{aq})** | **Activity Retention** |
|---|---|---|---|---|
| | | | | |
| Guanine | 20 mM | 9.2×10⁹ | 1.4×10¹⁰ | 0 % |
| Uracil | 20 mM | 5.7×10⁹ | 1.5×10¹⁰ | 14.4 % |
| Histidine | 20 mM | 4.8×10⁹ | 5×10⁸ | 0 % |

### Example 4: Completely non-polar compounds, with high rates of reaction with both hydroxyl radicals and hydrated electrons, which conferred some protection of glucose oxidase.

Table 4 shows examples of non-polar compounds with good rates of reaction with both hydroxyl radicals and hydrated electrons. Their interaction with histidine, lysine and tyrosine is possible due to their hydrophobic nature and absence of positive charge. These compounds offered protection to glucose oxidase against the effects of gamma-radiation so that 12.3 % of the original activity could be recovered in the presence of purine and 49.2 % in the presence of 1,10-phenanthroline and 8.1 % in the presence of methoxyphenol.

**Table 4.**

| **Compound** | **Concentration** | **k(OH·)** | **k(e⁻_{aq})** | **Activity Retention** |
|---|---|---|---|---|
| | | | | |
| Purine | 20 mM | 2.1 × 10¹⁰ | 1.2 × 10¹⁰ | 12.3 % |
| 1,10-phenanthroline | < 20 mM | 7×10⁹ | 1.8 × 10¹⁰ | 49.2 % |
| Methoxyphenol | 20 mM | 3.2×10¹⁰ | 9.7×10⁹ | 8.1% |

### Example 5: Compounds consisting mainly of non-polar regions with an adjacent polar groups, with good (or unknown) rates of reaction with both hydroxyl radicals and hydrated electrons, which conferred some protection of glucose oxidase.

Table 5 shows the protecting effects of compounds with a good reaction rate with both hydroxyl radical and hydrated electron, whose molecules comprise predominantly non-polar moiety with one or two polar groups attached. All of these compounds offered some protection of glucose oxidase. N.B. The rate constants for phenoxyethanol were not available, but they are believed to be reasonably high (in line with those for other phenoxy- compounds).

**Table 5.**

| **Compound** | **Concentration** | **k(OH·)** | **k(e⁻_{aq})** | **Activity Retention** |
|---|---|---|---|---|
| | | | | |
| Phenoxyethanol | 20 mM | N/A | N/A | 13.7 % |
| Adenine | < 20 mM | 5.8×10⁹ | 9×10⁹ | 6.2 % |
| Cytosine | < 20 mM | 6.3×10⁹ | 1.3×10¹⁰ | 37.1% |

### Example 6: Single compounds consisting mainly of non-polar regions (typically with polar groups attached) carrying a negative charge, with high rates of reaction with hydroxyl radicals and (in some cases) with hydrated electrons, which conferred protection of glucose oxidase.

The compounds listed in Table 6 have satisfactory rates of reaction with hydroxyl radicals (The rate of reaction of biotin with hydrated electron is only known at pH 9 while the experiments in this work were carried out at pH 7, so the figure shown is of uncertain relevance). Some of the compounds (particularly tryptophan and thiamine) also have a very high rate of reaction with hydrated electrons. All of these compounds are believed to undergo effective interactions with histidine, lysine and tyrosine because of the extensive non-polar regions in their molecules and the presence of negative charge. All of these compounds offered some protection to glucose oxidase against the effects of gamma radiation, especially tryptophan, thiamine and biotin.

**Table 6.**

| **Compound** | **Concentration** | **k(OH·)** | **k(e⁻_{aq})** | **Activity Retention** |
|---|---|---|---|---|
| | | | | |
| Phenylalanine | 20 mM | 6.5 × 10⁹ | 1.2 × 10⁸ | 15.2 % |
| Tyrosine | < 20 mM | 1.3×10¹⁰ | 2.8×10⁸ | 5.8% |
| Methionine | 20 mM | 7.4×10⁹ | 4.5×10⁷ | 16.9% |
| Tryptophan | 20 mM | 1.3×10¹⁰ | 3.2×10⁹ | 60.3 % |
| Thiamine | 20 mM | 3.0×10⁹ | 3.4×10¹⁰ | 47.6 % |
| Biotin | 20 mM | 3.8×10⁹ | 5×10⁷(N.B.pH9) | 52.7% |

### Example 7: Complex anions, with high rates of reaction with either hydroxyl radicals or hydrated electrons, which conferred protection of glucose oxidase.

The compounds listed in Table 7 are complex anions containing relatively non-polar CN groups. Whilst the ferrocyanide anion (hexacyanoferrate (II), Fe(CN)₆⁴-) has a very high reaction rate with hydroxyl radicals the ferricyanide anion (hexacyanoferrate (III), Fe^{III}(CN)₆³⁻) has a high reaction rate with hydrated electrons. The other reaction rates (i.e. ferrocyanide with hydrated electron and ferricyanide with hydroxyl radical) were not available. The interaction with histidine, lysine and tyrosine is believed to be good due to the negative charge and non-polar parts of the molecules (CN groups). Both compounds offered protection to glucose oxidase through gamma irradiation. Whilst this is not surprising in case of ferrocyanide owing to the high rate of reaction with hydroxyl radical the interpretation is more difficult in the case of ferricyanide as its reaction rate with hydroxyl radical has not been found. Nevertheless, even if this reaction rate was low the result would still have a plausible explanation: The reaction of ferricyanide with hydrated electron (during the gamma irradiation) results in generation of ferrocyanide. This means that there will always be at least trace amounts of ferrocyanide in the irradiated ferricyanide samples that will ensure protection against the hydroxyl radical.

**Table 7.**

| **Compound** | **Concentration** | **k(OH·)** | **k(e⁻_{aq})** | **Activity Retention** |
|---|---|---|---|---|
| | | | | |
| Ferrocyanide | 20 mM | 1.1 × 10¹⁰ | N/A | 54.3 % |
| Ferricyanide | 20 mM | N/A | 3.1×10⁹ | 21.7 % |

### Example 8: Combination of two anions which conferred little or no protection.

Table 8 shows the effect of the combination of two anions on the protection of glucose oxidase. Combination of iodide and nitrite did not offer any protection of glucose oxidase in spite of the fact that these two compounds share high rates of reaction with both hydroxyl radicals and hydrated electrons. This is believed to be due to the absence of non-polar structures and to the direct competition of the anions for the positively charged binding site of histidine and lysine. Similarly, the detrimental effect of anion competition was observed in the combination of iodide and ferricyanide. Although some protection of glucose oxidase was achieved it was no better than that achieved by ferricyanide alone (see Table 7). In fact, the simultaneous presence of iodide made the glucose oxidase recovery worse.

**Table 8.**

| **Compound** | **Concentration** | **k(OH·)** | **k(e⁻_{aq})** | **Activity Retention** |
|---|---|---|---|---|
| | | | | |
| Iodide + | 20 mM + | 1.2×10¹⁰ | 2.4×10⁵ | 0 % |
| Nitrate | 20 mM | N/A | 9.7×10⁹ | |
| Iodide + | 20 mM + | 1.2×10¹⁰ | 2.4×10⁵ | 13.9% |
| Ferricyanide | 20 mM | N/A | 3.1 × 10⁹ | |

### Example 9: Combination of two compounds which conferred extra protection.

Table 9 shows the effects of various pairs of compounds on the glucose oxidase recovery after gamma-irradiation. All compounds shown in Table 9 have been listed in the previous examples as single compounds. Whilst some of them, namely nitrate and iodide (see Tables 1 and 2), failed completely to protect the enzyme on their own, others did offer a degree of protection (see Tables 3 to 7). However, a considerable protection of the enzyme could be achieved by careful combination of the compounds into pairs (see Table 9). The compounds were combined so that:
- At least one of them had a high reaction rate with hydroxyl radicals and at least one of them had a high reaction rate with hydrated electrons.
- At least one of the compounds contained a substantial non-polar region.
- At least one of the compounds carried a negative charge (in most cases both compounds were negatively charged, but the direct competition of charges was minimised due to the presence of substantial non-polar parts in at least one of the molecules). N.B. One of the combinations (methoxyphenol and mannitol) did not contain any negative charge under the conditions of the experiment.

Combining the compounds into pairs led to improved recovery of glucose oxidase compared with that achieved with the individual compounds. Whilst in some cases there was only a slight improvement over the effectiveness of the single compounds (e.g. with tryptophan/nitrate and methoxyphenol/mannitol), in most cases the improvement was very significant (e.g. methionine/ferricyanide, uracil/ferricyanide).

**Table 9.**

| **Compound** | **Concentratio n** | **k(OH·)** | **k(e⁻_{aq})** | **Activity Retention** |
|---|---|---|---|---|
| | | | | |
| Ferrocyanide + | 20 mM + | 1.1×10¹⁰ | N/A | 84.9 % |
| Ferricyanide | 20 mM | N/A | 3.1×10⁹ | |
| Phenylalanine + | 20 mM + | 6.5×10⁹ | 1.2×10⁸ | 78.3 % |
| Ferricyanide | 20 mM | N/A | 3.1×10⁹ | |
| Tyrosine + | 20 mM + | 1.3×10¹⁰ | 2.8×10⁸ | 73.1% |
| Ferricyanide | 20 mM | N/A | 3.1 ×10⁹ | |
| Phenylalanine + | 20 mM + | 6.5×10⁹ | 1.2×10⁸ | 80.8 % |
| Nitrate | 20 mM | N/A | 9.7×10⁹ | |
| Tyrosine + | 20 mM + | 1.3×10¹⁰ | 2.8×10⁸ | 22.0 % |
| Nitrate | 20 mM | N/A | 9.7×10⁹ | |
| Cytosine + | 20 mM + | 6.3 × 10⁹ | 1.3 × 10¹⁰ | 69.3 % |
| Iodide | 20 mM | 1 × 10¹⁰ | 2.4 × 10⁵ | |
| Uracil + | 20 mM + | 5.7 × 10⁹ | 1.5×10¹⁰ | 60.0 % |
| Iodide | 20 mM | 1×10¹⁰ | 2.4×10⁵ | |
| Cytosine + | 20 mM + | 6.3×10⁹ | 1.3 × 10¹⁰ | 65.6 % |
| Ferrocyanide | 20 mM | 1.1×10¹⁰ | N/A | |
| Uracil + | 20 mM + | 5.7×10⁹ | 1.5×10¹⁰ | 67.9 % |
| Ferrocyanide | 20 mM | 1.1 ×10¹⁰ | N/A | |
| Cytosine + | 20 mM + | 6.3×10⁹ | 1.3×10¹⁰ | 86.2 % |
| Ferricyanide | 20 mM | N/A | 3.1 ×10⁹ | |
| Uracil + | 20 mM + | 5.7×10⁹ | 1.5×10¹⁰ | 81.6 % |
| Ferricyanide | 20 mM | N/A | 3.1×10⁹ | |
| Methionine + | 20 mM + | 7.4×10⁹ | 4.5×10⁷ | 81.8 % |
| Ferricyanide | 20 mM | N/A | 3.1 ×10⁹ | |
| Tryptophan + | 20 mM + | 1.3×10¹⁰ | 3.2×10⁹ | 72.4 % |
| Nitrate | 20 mM | N/L | 9.7×10⁹ | |
| Methoxyphenol | 20 mM + | 3.2×10¹⁰ | 9.7×10⁹ | 51.8 % |
| + Iodide | 20 mM | 1×10¹⁰ | 2.4×10⁵ | |
| Methoxyphenol | 20 mM + | 3.2×10¹⁰ | 9.7×10⁹ | 21.0 % |
| + Mannitol | 20 mM | 2.5×10⁹ | 7 × 10⁶ | |
| Thiamine + | 20 mM + | 3 × 10⁹ | 3.4 × 10¹⁰ | 83.5% |
| Iodide | 20 mM | 1 × 10¹⁰ | 2.4 × 10⁵ | |
| Thiamine + | 20 mM + | 3 × 10⁹ | 3.4 × 10¹⁰ | 71.1 % |
| Nitrate | 20 mM | N/A | 9.7 × 10⁹ | |

### Example 10: Combinations of compounds, with low radical reaction rates, which conferred little or no protection.

Table 10 shows the effects of various pairs of compounds on the glucose oxidase recovery. The combinations were selected so that the compounds did not share a high rate of reaction with both free radicals generated in gamma-irradiated aqueous solutions. So, whilst some pairs did not share a high reaction rate with either of the free radicals (e.g. proline/glycine, alanine/glycine), other pairs shared a high reaction rate with only one of the crucial free radicals (hydroxyl radical or hydrated electron). Most of the pairs did not give any protection to glucose oxidase at all. One pair (proline/iodide) gave a very slight protective effect (< 4%). In the case of proline/mannitol and proline/iodide the presence of positive charge on the proline side chain further contributes to the poor protective effect. Similarly, in the case of alanine/iodide and alanine/mannitol the absence of substantial non-polar regions might be an additional contributing factor to the poor overall protective effect.

**Table 10.**

| **Compound** | **Concentration** | **k(OH.)** | **k(e⁻_{aq})** | **Activity Retention** |
|---|---|---|---|---|
| | | | | |
| Alanine + | 20 mM + | 4.3×10⁸ | 1.2 × 10⁷ | 0 % |
| Iodide | 20 mM | 1×10¹⁰ | 2.4×10⁵ | |
| Alanine + | 20 mM + | 4.3×10⁸ | 1.2×10⁷ | 0 % |
| Mannitol | 20 mM | 2.5×10⁹ | 7×10⁶ | |
| Proline + | 20 mM + | 3.1×10⁸ | 2×10⁷ | 3.9% |
| Iodide | 20 mM | 1×10¹⁰ | 2.4×10⁵ | |
| Proline + | 20 mM + | 3.1×10⁸ | 2×10⁷ | 0 % |
| Mannitol | 20 mM | 2.5×10⁹ | 7×10⁶ | |
| Proline + | 20 mM + | 3.1×10⁸ | 2×10⁷ | 0% |
| Glycine | 20 mM | 1.7×10⁷ | 1×10⁷ | |
| Alanine + | 20 mM + | 4.3 × 10⁸ | 1.2 × 10⁷ | 0 % |
| Glycine | 20 mM | 1.7 × 10⁷ | 1 × 10⁷ | |
| Glycine + | 20 mM + | 1.7 × 10⁷ | 1 × 10⁷ | 0 % |
| Nitrate | 20 mM | N/A | 9.7×10⁹ | |

### Example 11: Combinations of compounds, with satisfactory radical reaction rates, that fail to protect the enzyme due to their unfavourable molecular structure.

Table 11 lists pairs of compounds that share a high rate of reaction with both hydroxyl radicals and hydrated electrons. Nevertheless, their ability to protect glucose oxidase against the effects of gamma radiation is compromised by their molecular structure. Thus, in the case of nitrate/mannitol the lack of protection was due to the absence of non-polar regions whereas the combinations including guanine are believed to have failed because of the positive charge on the guanine ring. See Table 8 for comparison.

**Table 11.**

| **Compound** | **Concentration** | **k(OH·)** | **k(e⁻_{aq})** | **Activity Retention** |
|---|---|---|---|---|
| | | | | |
| Nitrate + | 20 mM + | N/A | 9.7 × 10⁹ | 0 % |
| Mannitol | 20 mM | 2.5 × 10⁹ | 7×10⁶ | |
| Guanine + | 20 mM + | 9.2×10⁹ | 1.4×10¹⁰ | 0 % |
| Iodide | 20 mM | 1×10¹⁰ | 2.4×10⁵ | |
| Guanine + | 20 mM + | 9.2×10⁹ | 1.4×10¹⁰ | 0 % |
| Mannitol | 20 mM | 2.5×10⁹ | 7 ×10⁶ | |
| Guanine + | 20 mM + | 9.2×10⁹ | 1.4×10¹⁰ | 0 % |
| Glycine | 20 mM | 1.7×10⁷ | 1×10⁷ | |

### Example 12: Protection of catalase using single compounds.

Table 12 shows the protective effect of selected single compounds on the activity of the enzyme catalase during gamma irradiation. In the absence of the protective compounds (i.e. protein dissolved directly in phosphate buffer, 50 mM, pH 6) no enzyme activity was recovered after the sample irradiation. The presence of purine, tryptophan or lactate in the buffer solution resulted in a degree of catalase activity preservation. The activity recovery was relatively small (although clearly discernable from activity in the control sample) in the case of purine and more substantial in the case of tryptophan and lactate. N.B. Two sets of rate constants are given in Table 12 for lactate because lactate is partially protonated at the pH employed and exists as a mixture of lactic acid and lactate anion.

**Table 12.**

| **Compound** | **Concentration** | **k(OH·)** | **k(e⁻_{aq})** | **Activity Retention** |
|---|---|---|---|---|
| | | | | |
| Buffer only (control) | | | | 0 % |
| Purine | 20 mM | 2.1×10¹⁰ | 1.2×10¹⁰ | 7.4 % |
| Tryptophan | 20 mM | 9.2×10⁹ | 1.4×10¹⁰ | 44.7 % |
| Lactic acid + Lactate anion | 320 mM | 1.6×10¹⁰ 4.3×10⁸ | 1.0×10⁷ 7.0×10⁹ | 37.8 % |

### Example 13: Protection of anti-hCG monoclonal antibodies using single compounds.

Protection effect was studied of selected individual compounds on the activity recovery of two different monoclonal antibodies subjected to gamma irradiation. Both of the monoclonal antibodies were anti-hCG (i.e. recognising human chorionic gonadotropin). Whilst the first antibody was expressed to recognise an epitope on the α-chain of hCG the second antibody was expressed to recognise an epitope on the β-chain of hCG. The protective effect of selected compounds on the activity recovery of the first antibody is shown in Table 13. The protective effect of selected compounds on the activity recovery of the second antibody is shown in Table 14.

Incorporation of the selected compounds conferred considerable protection of the two antibodies in aqueous solutions. Whilst in the absence of the protective compounds (i.e. dissolved directly in water) no activity was recovered after the sample irradiation the presence of either purine, lactate, nicotinate or tryptophan resulted in a degree of activity preservation.

**Table 13.**

| **Compound** | **Concentration** | **k(OH·)** | **k(e⁻_{aq})** | **Activity Retention** |
|---|---|---|---|---|
| | | | | |
| Water only (control) | | | | 0 % |
| Nicotinate anion | 80 mM | 2.5×10⁹ | 1.2×10¹⁰ | 53 % |
| Lactic acid + | 320 mM | 1.6×10¹⁰ | 1.0×10⁷ | 83 % |
| Lactate anion | | 4.3×10⁸ | 7.0×10⁹ | |
| Tryptophan | 5 mM | 9.2×10⁹ | 1.4×10¹⁰ | 100 % |

**Table 14.**

| **Compound** | **Concentration** | **k(OH·)** | **k(e⁻_{aq})** | **Activity Retention** |
|---|---|---|---|---|
| | | | | |
| Water only (control) | | | | 0 % |
| Nicotinate anion | 80 mM | 2.5×10⁹ | 1.2×10¹⁰ | 100 % |
| Lactic acid + | 320 mM | 1.6×10¹⁰ | 1.0×10⁷ | 100 % |
| Lactate anion | | 4.3×10⁸ | 7.0×10⁹ | |
| Tryptophan | 5 mM | 9.2×10⁹ | 1.4×10¹⁰ | 100 % |
| Purine | 80 mM | 2.1×10¹⁰ | 1.2×10¹⁰ | 80 % |

### Example 14: Protection of horseradish peroxidase using single compounds.

Protection effect was studied of selected individual compounds on the activity recovery of aqueous horseradish peroxidase subjected to gamma irradiation. Incorporation of the selected compounds conferred considerable protection of the enzyme in aqueous solutions (Table 15). Whilst in the absence of the protective compounds (i.e. dissolved directly in water) almost no activity was recovered after the sample irradiation the presence of either purine, lactate or tryptophan resulted in a degree of activity preservation.

**Table 15.**

| **Compound** | **Concentration** | **k(OH·)** | **k(e⁻_{aq})** | **Activity Retention** |
|---|---|---|---|---|
| | | | | |
| Water only (control) | | | | 1.4 % |
| Lactic acid + | 320 mM | 1.6×10¹⁰ | 1.0×10⁷ | 14.2 % |
| Lactate anion | | 4.3×10⁸ | 7.0×10⁹ | |
| Tryptophan | 5 mM | 9.2×10⁹ | 1.4 × 10¹⁰ | 84.9 % |
| Purine | 80 mM | 2.1 × 10¹⁰ | 1.2×10¹⁰ | 67.6 % |

### Example 15: Protection of papain using single compounds.

Protection effect was studied of selected individual compounds on the activity recovery of aqueous papain subjected to gamma irradiation. Incorporation of the selected compounds conferred considerable protection of the enzyme in aqueous solutions (Table 16). Whilst in the absence of the protective compounds (i.e. dissolved directly in water) almost no activity was recovered after the sample irradiation the presence of either lactate, tryptophan, nicotinate anion or combination of phenylalanine and nitrate resulted in a degree of activity preservation.

**Table 16.**

| **Compound** | **Concentration** | **k(OH·)** | **k(e⁻_{aq})** | **Activity Retention** |
|---|---|---|---|---|
| | | | | |
| Water only (control) | | | | 0 % |
| Lactic acid + | 320 mM | 1.6×10¹⁰ | 1.0×10⁷ | 9.9 % |
| Lactate anion | | 4.3×10⁸ | 7.0×10⁹ | |
| Tryptophan | 5 mM | 9.2×10⁹ | 1.4×10¹⁰ | 28.5 % |
| Nicotinate anion | 80 mM | 2.5×10⁹ | 1.2×10¹⁰ | 43.0 % |
| Phenylalanine | 100 mM + | 6.5×10⁹ | 1.2×10⁸ | 9.0 % |
| + Nitrate | 20 mM | N/A | 9.7×10⁹ | |

## Claims

1. A method of sterilising a protein in an aqueous environment, comprising (a) bringing the protein into contact with a protective compound or combination of protective compounds having the following characteristics:
(i) a rate of reaction with hydroxyl radicals greater than 5 x 10⁸ L mol⁻¹ s⁻¹; and
(ii) a non-polar region,
excluding the case where the protein is an enzyme and the protective compound(s) comprise a source of lactate ions and a source of zinc ions and/or a source of ammonium ions and excluding guanine and histidine as protective compounds; and (b) exposing the protein and protective compound(s) to ionising radiation.

2. A method of stabilising a protein in an aqueous environment during sterilisation by exposure to ionising radiation, comprising bringing the protein into contact with a protective compound or combination of protective compounds having the following characteristics:
(i) a rate of reaction with hydroxyl radicals greater than 5 x 10⁸ L mol⁻¹ s⁻¹; and
(ii) a non-polar region,
excluding the case where the protein is an enzyme and the protective compound(s) comprise a source of lactate ions and a source of zinc ions and/or a source of ammonium ions and excluding guanine and histidine as protective compounds.

3. A composition comprising a protein in an aqueous environment and a protective compound or combination of protective compounds having the following characteristics:
(i) a rate of reaction with hydroxyl radicals greater than 5 x 10⁸ L mol⁻¹ s⁻¹; and
(ii) a non-polar region,
excluding the case where the protein is an enzyme and the protective compound(s) comprise a source of lactate ions and a source of zinc ions and/or a source of ammonium ions, excluding the case where the protein is bilirubin oxidase and the protective compound is tryptophan, excluding the case where the enzyme is lactate dehydrogenase and the protective compound is malic acid salt, excluding the case where the enzyme is lactate dehydrogenase and the protective compound is succinic acid salt, and excluding guanine and histidine as protective compounds.

4. A composition according to claim 3, which has been sterilised by exposure to ionising radiation.

5. A composition according to claim 4, in microbiologically sterile condition.

6. The invention of any one of the preceding claims, wherein the protective compound or combination of protective compounds satisfies the requirement of having a rate of reaction with hydroxyl radicals greater than 10⁹ L mol⁻¹ s⁻¹.

7. The invention of any one of the preceding claims, wherein the protective compound or combination of protective compounds satisfy the requirement of having a non-polar region that does not have a positive charge directly on the non-polar region.

8. The invention of any one of the preceding claims, wherein the protective compound or combination of protective compounds satisfy the requirement of having a negative charge at neutral pH.

9. The invention of any one of the preceding claims, wherein one or more compounds of the protective compound or combination of protective compounds satisfy the requirement of having a rate of reaction with hydrated electrons greater than 10⁸ L mol⁻¹ s⁻¹, preferably greater than 5 x 10⁸ L mol⁻¹ s⁻¹, more preferably greater than 10⁹ L mol⁻¹ s⁻¹.

10. The invention of any one of the preceding claims, wherein the non-polar region is an aliphatic chain, heterocyclic or aromatic ring structure that is capable of forming non-covalent hydrophobic bonds with the side chains of hydrophobic amino acids such as tryptophan, lysine or histidine.

11. The invention of any one of the preceding claims, wherein one or more of the protective compound or compounds possesses a non-polar region to which one or a limited number of polar groups is attached.

12. The invention of any one of the preceding claims, wherein the protective compound(s) are used at a concentration in the range 1 mM to 1 M, preferably 1 mM to 200 mM, most preferably 5 mM to 100 mM.
